# EUROPÄISCHE PATENTSCHRIFT

(11) **EP 1 265 069 B1**
(45) Veröffentlichungstag und Bekanntmachung des Hinweises auf die Patenterteilung: **17.09.2003**
(21) Anmeldenummer: 01113712.2
(22) Anmeldetag: 05.06.2001
(51) Int. Cl.: G01N 33/50, G01N 33/53, C12Q 1/68, G06F 19/00

(54) **Diagnostisches Verfahren auf der Grundlage von Lipidmessparameter-Modulations/Effektorquotientenprofilen**
Diagnostic method based on the measurement of lipid inflammation mediators' modulation/effector ratio profiles
Procédé de diagnostique utilisant la détermination pour les lipides mediateurs d'inflammation des profiles du rapport modulateur/effecteur

(43) Veröffentlichungstag der Anmeldung: 11.12.2002
(73) Patentinhaber: Baenkler, Hanns-Wolf, 91074 Herzogenaurach (DE); Schäfer, Dirk, 91301 Forchheim (DE)
(72) Erfinder: Baenkler, Hanns-Wolf, 91074 Herzogenaurach (DE); Schäfer, Dirk, 91301 Forchheim (DE)
(74) Vertreter: Stürken, Joachim

(56) Entgegenhaltungen:
- SCHAEFER D ET AL: "EFFECT OF PROSTAGLANDIN E2 ON EICOSANOID RELEASE BY HUMAN BRONCHIALBIOPSY SPECIMENS FROM NORMAL AND INFLAMED MUCOSA" THORAX, BMJ PUBLISHING GROUP, GB, Bd. 51, Nr. 9, 1996, Seiten 919-923, XP001017896 ISSN: 0040-6376
- MOHAMMADIAN P, SCHAEFER D, HUMMEL T, BAENKLER HW, KOBAL G: "A reversible inflammation model in man" NAUNYN-SCHMIEDEBERG'S ARCHIVES OF PHARMACOLOGY, Bd. 353, Nr. 4 suppl, 1996, Seite r150 XP001018295
- BAENKLER H W ET AL: "EICOSANOIDS FROM BIOPSY OF NORMAL AND POLYPOUS NASAL MUCOSA" RHINOLOGY, LEIDEN, NL, Bd. 34, Nr. 3, 1996, Seiten 166-170, XP001017931 ISSN: 0300-0729
- SCHAEFER D ET AL: "DYNAMICS OF EICOSANOIDS IN PERIPHERAL BLOOD CELLS DURING BRONCHIAL PROVOCATION IN ASPIRIN-INTOLERANT ASTHMATICS" EUROPEAN RESPIRATORY JOURNAL, MUNKSGAARD INTERNATIONAL PUBLISHERS, COPENHAGEN, DK, Bd. 13, Nr. 3, März 1999 (1999-03), Seiten 638-646, XP001017933 ISSN: 0903-1936
- BOZZA P T ET AL: "MECHANISMS OF FORMATION AND FUNCTION OF EOSINOPHIL LIPID BODIES: INDUCIBLE INTRACELLULAR SITES INVOLVED IN ARACHIDONIC ACID METABOLISM" MEMORIAS DO INSTITUTO OSWALDO CRUZ, RIO DE JANEIRO, BR, Bd. 92, Nr. SUPPL 2, 1997, Seiten 135-140, XP001017863 ISSN: 0074-0276
- POMPEIA C ET AL: "FATTY ACIDS AND THE IMMUNE SYSTEM" REVISTA BRASILEIRA DE CIENCIAS FARMACEUTICAS - BRAZILIAN JOURNAL OF PHARMACEUTICAL SCIENCES, FACULDADE DE CIENCIAS FARMACEUTICAS, SAO PAULO,, BR, Bd. 35, Nr. 2, 1999, Seiten 165-194, XP001018131 ISSN: 1516-9332

## Beschreibung

Die Erfindung betrifft ein Verfahren zur Sicherung oder zum Ausschluß von Risikokonstellationen, Krankheitszuständen oder Prädispositionen dafür, sowie ein Verfahren zur Überwachung des Verlaufs von Therapien und zum Auffinden von Wirkstoffen zur Behandlung von Krankheitszuständen und zum Auffinden von Wirkstoffen, die einen solchen Krankheitszustand auslösen können, auf der Grundlage von Lipidmeßparameter-Modulations/-Effektorquotientenprofilen. Ferner betrifft die vorliegende Erfindung ein Messinstrument zur Durchführung der genannten Verfahren.

Das Vorkommen von Lipiden/Eicosanoiden im menschlichen Körper und ihre Bedeutung für potentiell viele Körperfunktionen des Menschen wurde bereits vor über 100 Jahren beschrieben. Ihre Biosynthese sowie deren Beeinflussung durch komplexe als auch reine chemische Verbindungen ist bereits seit dem Altertum bekannt (z.B. die schmerzlindernde Wirkung des Bruchweidenextraktes) und wurde biochemisch seit Mitte des 20. Jahrhunderts immer detaillierter aufgeklärt (1-15). Aber auch das Vorkommen der Lipide/Eicosanoide und ihre einflußnehmende Wirkung im Tierreich (auch in Amphibien, Insekten und Mikroorganismen) und den Pflanzen ist bekannt (16-19). Weiterhin wachsen die Erkenntnisse über die verschiedenen Lipid/Eicosanoidrezeptoren und möglicher Subtypen zunehmend. Auch sind z.T. die Gene und ihre chromosomalen Lokalisationen für einige Enzyme des Eicosanoidmetabolismus und der Eicosanoidrezeptoren bekannt (20-26).

Der Nachweis von Lipiden/Eicosanoiden erfolgt zur Zeit mittels verschiedener physikalisch-chemischer und immunologischer Techniken (z.B. durch Gaschromatographie, Hochdruckflüssigkeitschromatographie (HPLC), Dünnschichtchromatographie, Radioimmunoassays (RIA), Enzymimmunoassays (EIA)). Diese Methoden unterscheiden sich hinsichtlich der Nachweisempfindlichkeit und Auftrennmöglichkeit verschiedener Lipide/Eicosanoide während des Meßvorgangs sowie des maximalen Probendurchsatzvolumens (27).

Der Nachweis bzw. die Bestimmung von Enzymen, die an der Synthese bzw. dem Abbau von Lipiden/Eicosanoiden beteiligt sind, erfolgt meist nach gelelektrophoretischer Auftrennung der zellulären Proteine durch anschließende Markierung mittels spezifischer Antikörper oder auf intakten Zellen mit radioaktiv oder anderweitig markierten Lipiden/Eicosanoiden bzw. Rezeptorliganden (Agonisten/Antagonisten). Auch ist ein immuncytologischer Nachweis der Enzyme bzw. Rezeptoren mittels immuncytologischer Methoden unter Verwendung geeigneter monoklonaler oder polyklonaler Antikörper möglich. Enzymatische Aktivitätsnachweise bzw. -bestimmungen erfolgen direkt durch Messung des Abbaus geeigneter Enzymsubstrate oder indirekt durch den Nachweis sekundär gebildeter Metaboliten (20, 21, 24, 37, 29-30).

Der Nachweis bzw. die Bestimmung von mRNA für die Enzyme und Rezeptoren, die an der Synthese bzw. dem Abbau von Lipiden/Eicosanoiden oder an deren Bindung beteiligt sind, kann z.B. mittels RT-PCR (engl. »reverse transcriptase polymerase chain reaction«) oder »northern blotting« erfolgen.

Die Lipide/Eicosanoide wurden bisher für klinisch-diagnostische Zwecke nicht bzw. nicht in breitem Umfang genutzt (31,32), obwohl Analyseverfahren zur Gesamtbestimmung oder auch zur selektiven Bestimmung von bestimmten Eicosanoiden bekannt sind (21-30). Nur für einzelne Eicosanoide, die Peptid-Leukotriene, befindet sich ein Testsystem auf dem Markt (»CAST-Elisa«), das zum Nachweis erhöhter Peptid-Leukotrienspiegel nach in-vitro-Stimulation mit Allergenen in Kombination mit generell zellaktivierenden Substanzen (z.B. Komplementfaktoren oder Cytokinen) dient. Aus der Menge der freigesetzten Lipide/Eicosanoide, d.h. der Peptid-Leukotriene, soll der Grad der Sensibilisierung der untersuchten (allergischen) Patienten abzuleiten sein (32). Auch über die Veränderung der Lipid/Eicosanoidspiegel bei verschiedenen Krankheitsbildern ist berichtet worden (33-37).

Die verschiedenen Autoren weisen in ihren Arbeiten auf die Meßbarkeit von veränderten Lipid/Eicosanoidmengen in den von ihnen untersuchten Proben hin, und daß viele der untersuchten Krankheitsbilder mit erhöhten Lipid/Eicosanoidspiegeln einhergehen. Grundsätzlich wird aber nicht auf ein Lipid/Eicosanoidmuster oder -profil, welches sich gegenüber einem Normalzustand verändert hat, eingegangen. Auch wird meist nur der Ist-Zustand von Lipiden/Eicosanoiden bestimmt. Es wird jedoch nicht z.B. die Auswirkung einer Modulation (Stimulation/Inhibition) der Eicosanoidsynthese des biologischen Materials in vitro für diagnostische Zwecke in Erwägung gezogen (mit Ausnahem des bereits erwähnten CAST-ELISA). Enzyme und Rezeptoren des Lipid/Eicosanoidstoffwechsels wurden nach bisherigem Wissensstand nicht für diagnostische Zwecke, weder im Ist-Zustand noch im modulierten Zustand, herangezogen.

Aus diesem Stand der Technik ergibt sich die Aufgabe der Erfindung.

Die Erfindung betrifft somit ein Verfahren zur Sicherung oder zum Ausschluß von Krankheitszuständen oder Prädispositionen dafür auf der Grundlage von Lipidmeßparameter-Modulations/Effektorquotientenprofilen, bei dem
(a) eine Probe aus einem zu untersuchenden Organismus entnommen wird,
(b) die Probe aus dem zu untersuchenden Organismus in soviele gleiche Teilproben aufgeteilt wird, daß für jeden Lipidmeßparameter (eine Definition dieses Begriffs wird weiter unten gegeben) A, B, C ... ein Nullwert A₀, B₀, C₀, ... in Abwesenheit eines modulierenden Effektors (bzw. Mediators); ein Wert für eine Leitsubstanz (Leitwert) Aₘₐₓ, Bₘₐₓ, Cₘₐₓ, ... in Gegenwart eines modulierenden Effektors (bzw. Mediators) (Leitwert, da es auch Fälle gibt (z.B. bei Verwendung eines chemotaktischen Peptids, wie fMLP, N-Formyl-met-Leu-Phe-Phe), bei denen der Meßwert für den weiteren modulierenden Effektor (wie im folgenden angegeben) höher ist als der »Leitwert«); und mindestens ein Wert für eine weitere (in der Regel (aus den oben erläuterten Gründen) intermediäre, d.h. sich unter oder zwischen dem Nullwert und dem Leitwert befindliche) Modulation A₂, B₂, C₂, ... in Gegenwart eines weiteren modulierenden Effektors (bzw. Mediators) gemessen werden kann,
(c) für jeden Lipidmeßparameter A, B, C, ... der Probe aus dem zu untersuchenden Organismus
   (i) die Quotienten der Meßwerte Aₘₐₓ/A₀, A₂/A₀; Bₘₐₓ/B₀, B₂/B₀; Cₘₐₓ/C₀, C₂/C₀; ... gebildet und anschließend die für den zu untersuchenden Organismus erhaltenen Werte durch die entsprechenden Werte einer oder mehrerer Normgruppe(n) dividiert werden, wodurch sich normierte Modulationsquotienten ergeben, die in ihrer Gesamtheit ein normiertes Modulationsquotientenprofil für den zu untersuchenden Organismus bilden, und
   (ii) aus den Nullwerten A₀, B₀, C₀ ... Quotienten A₀/B₀, B₀/A₀, A₀/C₀, C₀/A₀, B₀/C₀, C₀/B₀ ... in beliebiger Kombination gebildet werden; aus den Werten für eine maximale Modulation mit einer Leitsubstanz Aₘₐₓ, Bₘₐₓ, Cₘₐₓ ... Quotienten Aₘₐₓ/Bₘₐₓ, Bₘₐₓ/Aₘₐₓ, Aₘₐₓ/Cₘₐₓ, Cₘₐₓ/Aₘₐₓ, Bₘₐₓ/Cₘₐₓ, Cₘₐₓ/Bₘₐₓ ... in beliebiger Kombination gebildet werden; und aus den Werten für mindestens eine weitere Modulation A₂, B₂, C₂ ... Quotienten A₂/B₂, B₂/A₂, A₂/C₂, C₂/A₂, B₂/C₂, C₂/B₂ ... in beliebiger Kombination gebildet werden; und anschließend die für den zu untersuchenden Organismus erhaltenen Werte durch die entsprechenden Werte einer oder mehrerer Normgruppe(n) dividiert werden, wodurch sich normierte Effektorquotienten ergeben, die in ihrer Gesamtheit ein normiertes Effektorquotientenprofil für den zu untersuchenden Organismus bilden,
und
(d) eine Risikokonstellation, ein Krankheitszustand oder eine Prädispositionen dafür durch den Vergleich der normierten Modulations- und Effektorquotientenprofile des zu untersuchenden Organismus mit denen einer entsprechenden Untersuchungsgruppe, bei der die interessierende Risikokonstellation, der interessierende Krankheitszustand oder die interessierende Prädisposition vorliegt, gesichert oder ausgeschlossen wird.

Es ist klar, daß bei der Berechnung der Lipidmeßparameter-Modulations/Effektorquotienten die Fehlerfortpflanzung berücksichtigt wird. Ferner läßt sich grob sagen, daß ein Lipidmeßparameter-Modulations/Effektorquotient auffällig ist, wenn er etwa 5 - 10 % höher oder niediger als der jeweilige Normwert ist. Als weiteres Entscheidungskriterium zur Abgrenzung Normal/Auffällig kann auch die zweifache Standardabweichung 2σ des jeweiligen normalen Lipidmeßparameter-Modulations/Effektorquotienten dienen. Ist der betreffende Wert gegenüber dem Normwert ± 2σ verändert, wird er als auffällig angesehen.

Es ist klar, das der Begriff »Normgruppe« bzw. »Untersuchungsgruppe« relativ ist. Die »Normgruppe« kann auch durch den zu untersuchenden einzelnen Organismus selber oder durch Teile desselben (z.B. Gewebe, Körperflüssigkeiten) gebildet werden, z.B. wenn Meßwerte im gesunden Zustand später zur Normierung von Meßwerten in einem akuten Krankheitszustand verwendet werden, oder auch einfach nur um festzustellen, ob irgendwelche Veränderungen eingetreten sind. In analoger Weise gilt dies auch für die »Untersuchungsgruppe«, z.B. wenn Werte im akuten Krankheitszustand nach erfolgter Therapie mit dem Ist-Zustand verglichen werden. Es ist ferner klar, daß die Meßwerte für die Normgruppen und die Untersuchungsgruppen oder die Meßwerte auch nur für einen zu untersuchenden einzelnen Organismus im Normalzustand bzw. im Zustand akuter Erkrankung auch in einer Datenbank gespeichert und bei Bedarf abgerufen und zur Normierung bzw. zum Vergleich herangezogen werden können. Sollten derartige Meßwerte nicht vorliegen, können sie ohne weiteres unter Anwendung des erfindungsgemäßen Verfahrens in entsprechenden »normalen« und »pathologischen« Zuständen oder mit entsprechenden »Normgruppen« und »Untersuchungsgruppen« erhoben werden.

Die Bestimmung von Lipidmeßparametern erfolgte bisher nahezu ausschließlich für einzelne Fragestellungen und meist ohne klinisch-diagnostischen Bezug. Sofern überhaupt eine diagnostische Fragestellung besteht (z.B. beim »CAST-ELISA« der Fa. Bühlmann-Laboratories), wird aber nur ein Lipidmeßparameter (hier die Peptid-Leukotriene) bestimmt oder z.B. nur die Grundmenge an Eicosanoid 1 und 2 und evtl. 3 oder Enzym 1 und 2 etc. oder die Synthese von Eicosanoid 1 erfaßt (32). Nach dem erfindungsgemäßen Verfahren wird hingegen insbesondere das Verhältnis verschiedener Lipidmeßparameter zueinander bestimmt (sog. Balancen) und klinisch-diagnostisch ausgewertet, die zu einer Einstufung in Risikonstellationen führt.

Die nach dem erfindungsgemäßen Verfahren erhaltenen Lipidmeßparameter-Modulations/Effektorquotientenprofile ermöglichen die Differenzierung von Krankheitsbildern, das »Monitoring« von Therapien, die Abschätzung von anti-inflammatorischen/pro-inflammatorischen Effekten von Stoffen, Substanzen bzw. Materialien und letztlich auch die inflammatorische Risikoabschätzung bekannter und unbekannter Stoffe/-Stoffgruppen in vitro. Mit Hilfe neuer Technologien (Micro-Multi-Array-Techniken) könnten nunmehr auch kleinste Probenmengen nach dem geschilderten Verfahren analysiert und daraus wichtige Rückschlüsse gezogen werden.

So kann z.B. der Verlauf einer Desensibilisierung gegen Allergene oder eine Desaktivierung gegenüber Acetylsalicylsäure (Aspirin®) direkt gemessen werden, ohne das Wohl des Patienten zu beeinträchtigen. Sowohl die Modulation als auch die Messung finden ex-vivo statt. Eine Messung in vivo ist nicht nötig. Gegenüber dem Stand der Technik beruht das erfindungsgemäße Verfahren auf der Informationsgewinnung durch den Vergleich mindestens zweier Lipidmeßparameter im modulierten (stimulierten/inhibierten) und unmoduliertem Zustand. Eine Risikokonstellation kann nicht über einen Lipidmeßparameter alleine zuverlässig bestimmt werden (beim CAST-ELISA (32) wird z.B. nur 1 Lipidmeßparameter bestimmt), sondern nur durch die nach dem erfindunggemäßen Verfahren erhaltenen Lipidmeßparameter-Modulations / Effektorquotientenprofile, die z.B. das Verhältnis von z.B. Prostaglandinen zu Leukotrienen berücksichtigen.

Weitere vorteilhafte oder bevorzugte Ausführungsformen der Erfindung sind Gegenstand der Unteransprüche.

Nach einer Ausführungsform des erfindungsgemäßen Verfahrens werden in Schritt (b) die Lipidmeßparameter unter Meßparametern für ungesättigte Fettsäuren, Abbau- und Syntheseenzyme für ungesattigte Fettsäuren sowie dafür kodierende Nukleinsäuren (mRNA), und unter solchen für Rezeptoren für ungesättigte Fettsäuren sowie dafür kodierende Nukleinsäuren (mRNA) ausgewählt.

Beispielsweise werden die ungesättigten Fettsäuren unter dem Thrombozyten-aktivierenden Faktor (= PAF, engl. »platelet activating factor«) (12) und den Eicosanoiden, z.B. Peptid-Leukotrienen (= pLTs, z.B. LTC4, LTD4, LTE4), Prostaglandin E2 (PGE2), Thromboxan A2 und Thromboxan B2 (= TXB2), ausgewählt.

Nach einer weiteren Ausführungsform des erfindungsgemäßen Verfahrens wird in dem oben definierten Schritt (b) als maximal modulierender Effektor (Leitsubstanz) Arachidonsäure oder ein chemotaktisches Peptid wie z.B. fMLP verwendet.

Nach einer weiteren Ausführungsform des erfindungsgemäßen Verfahrens wird in dem oben definierten Schritt (b) als weiterer modulierender Effektor ein Stoff (z.B. Viren, Bakterien oder andere Organismen wie Hefen, Pilze oder Bestandteile davon, Chemikalien im weitesten Sinne wie Lösungsmittel oder Farbstoffe, Allergene im weitesten Sinne, insbesondere auch biologischen Ursprungs, Arzneimittel, Toxine, insbesondere auch biologischen Ursprungs) verwendet, der einen auffälligen Zustand, z.B. einen Krankheitszustand, hervorrufen kann oder an dessen Entstehung oder Entwicklung beteiligt ist.

Das erfindungsgemäße Verfahren eignet sich beispielsweise zur Sicherung oder zum Ausschluß der folgenden Krankheitszustände sowie zur Abschätzung einer Prädisposition für dieselben: Tumore, beispielsweise bronchiale Tumore, cystische Fibrose, Polyposis, z.B. Polyposis nasi et sinuum, Asthma bronchiale, Unverträglichkeiten/Intoleranz gegen Nahrungsmittel, Nahrungsmittelzusatzstoffe oder Arzneimittel, z.B. gegen Analgetika wie Acetylsalicylsäure (Aspirin®), Allergien wie Pollen-, Sporen-, Milben-, Wespen- und/oder Bienengiftallergie, etwa als allergisches Asthma, unterschiedliche Entzündungen wie Enzephalitis, Sinusitis, Rhinitis, Neurodermits, Morbus Crohn, Colitis ulcerosa, Diarrhöen, Infektbewältigung, z.B. an Schleimhäuten, z.B. zur Abwehr bakterieller, viraler oder mykotischer Elemente, z.B. bei bakterieller, viraler oder mykotischer Mucositis, bei Infektanfälligkeit, Gerinnungsstörungen, z.B. Thrombosen, Blutungen oder Thrombophilie.

Nach einer Ausführungsform des erfindungsgemäßen Verfahrens wird/werden zur Bestimmung der Lipidmeßparameter ein oder mehrere, gegebenenfalls markierte(s) *Eicosanoid(e)* oder der Farbstoff 9-Diethylamino-5H-benzo[alpha]phenoxazin-5-on (38-40) verwendet.

Nach einer Ausführungsform des erfindungsgemäßen Verfahrens werden zur Bestimmung der Lipidmeßparameter immobilisierte Sonden verwendet, die ausgewählt sind unter: Antikörpern oder funktionellen Fragmenten davon gegen Abbau- oder Syntheseenzyme von ungesättigten Fettsäuren oder gegen Rezeptoren für ungesättigte Fettsäuren; Nukleinsäuren, die an Nukleinsäuren hybridisieren, die für Abbau- oder Syntheseenzyme von ungesättigten Fettsäuren oder für Rezeptoren für ungesättigte Fettsäuren kodieren.

Eine Sonde ist ganz allgemein ein Erkennungsmolekül oder ein Rezeptor, das/der einen Liganden spezifisch erkennen und binden kann.

Unter einem »funktionellen« Fragment eines Antikörpers wird ein Antikörperfragment verstanden, das an ein Antigen binden kann, das aber nicht notwendigerweise auch immunogen sein muß.

Geeignete Antikörper werden unter polyklonalen, monoklonalen und Single-chain-Antikörpern ausgewählt.

Geeignete Nukleinsäuren werden unter cDNA, mRNA und Oligonukleotiden ausgewählt.

Vorzugsweise bilden die immobilisierten Sonden ein adressierbares Muster auf einer Oberfläche, wodurch ein sogenannter Biochip entsteht.

Die Erfindung betrifft ferner ein Verfahren zur Überwachung des Verlaufs von Therapien von auffälligen Zuständen (z.B. Krankheitszuständen) auf der Grundlage von Lipidmeßparameter-Modulations/Effektorquotientenprofilen, bei dem das oben definierte erfindungsgemäße Verfahren nach der Verabreichung/Applikation (d.h. das zu untersuchende Medikament wird den Probanden bzw. dem Organismus vor der Probennahme verabreicht) oder in Gegenwart (d.h. das zu untersuchende Medikament wird der Probe nach deren Entnahme aus dem Probanden bzw. Organismus zugesetzt) eines geeigneten Medikaments durchgeführt wird. Dieses Medikament kann als einer der »weiteren modulierenden Effektoren« im Sinne der Erfindung angesehen werden. Es können natürlich auch Medikamentenkombinationen verwendet werden.

Die Erfindung betrifft ferner ein Verfahren zum Auffinden von Wirkstoffen zur Behandlung von Krankheitszuständen auf der Grundlage von Lipidmeßparameter-Modulations/Effektorquotientenprofilen, bei dem das oben definierte erfindungsgemäße Verfahren nach der Verabreichung (d.h. der zu untersuchende Kandidatenwirkstoff wird dem Probanden/Organismus vor der Probennahme verabreicht) oder in Gegenwart (d.h. der zu untersuchende Kandidatenwirkstoff wird der Probe nach deren Entnahme aus dem Probanden/Organismus zugesetzt) eines geeigneten Kandidatenwirkstoff durchgeführt wird. Dieser Kandidatenwirkstoff kann als einer der »weiteren modulierenden Effektoren« im Sinne der Erfindung angesehen werden. Es können natürlich auch Kandidatenwirkstoffkombinationen verwendet werden.

Die Erfindung betrifft außerdem ein Verfahren zum Auffinden von Stoffen, die in der Lage sind, einen oben definierten Krankheitszustand auszulösen, auf der Grundlage von Lipidmeßparameter-Modulations/Effektorquotientenprofilen, bei dem das oben definierte erfindungsgemäße Verfahren nach der Verabreichung/Applikation (d.h. der zu untersuchende Stoff wird dem Probanden/Organismus vor der Probennahme verabreicht/ appliziert) oder in Gegenwart (d.h. der zu untersuchende Stoff wird der Probe nach deren Entnahme aus dem Probanden/Organismus zugesetzt) eines solchen Stoffes durchgeführt wird.

Die Erfindung betrifft ferner ein Meßinstrument zur Durchführung der oben definierten erfindungsgemäßen Verfahren, das eine Oberfläche aufweist, auf der die oben definierten Sonden immobilisiert sind.

Vorzugsweise bilden die Sonden ein adressierbares Muster auf der Oberfläche, wodurch ein sog. Biochip entsteht.

Im folgenden wird die Erfindung ohne Beschränkung und unter Bezugnahme auf konkrete Beispiele detaillierter veranschaulicht.

Allgemein betrifft die Erfindung ein Verfahren zur Sicherung oder zum Ausschluß von auffälligen Zuständen z.B. Krankheitszuständen oder Prädispositionen dafür, sowie ein Verfahren zur Überwachung des Verlaufs von Therapien und zum Auffinden von Wirkstoffen zur Behandlung von auffälligen Zuständen z.B. Krankheitszuständen, das auf der Ermittlung des spontanen, allgemein modulierbaren (d.h. induzierbaren bzw. aktivierbaren/inhibierbaren) oder des speziell provozierten Verhaltens von Geweben und Zellen bezüglich der Synthese, Abgabe, Umsetzung oder des Abbaus von Lipiden wie Eicosanoiden, deren Rezeptoren oder Abbau/Syntheseenzyme, und zwar sowohl absolut als auch im gegenseitigen Verhältnis, beruht.

Im Gegensatz zum Stand der Technik wird nach dem erfindungsgemäßen Verfahren zum einen der Grundzustand (der native, nicht modulierte Zustand, der dem Nullwert entspricht) und zum anderen der modulierte Zustand (z.B. bei Stimulation mit einer Leitsubstanz und mindestens einem weiteren Modulator) von Lipidmeßparametern, z.B. der synthetisierten Lipide/Eicosanoide, der Enzyme der Lipid/Eicosanoidsynthese und/oder der Lipid/Eicosanoidrezeptoren, untersucht. Weiterhin werden mindestens zwei verschiedene Lipidmeßparameter bestimmt, z.B. Eicosanoide, Eicosanoidenzyme und/oder Eicosanoid-Rezeptoren der selben Probe gleichzeitig analysiert. Hierdurch wird nicht der statische (Grund/Ist)-Zustand erfaßt, sondern zusätzlich die Dynamik/Modulierbarkeit des zu untersuchenden Systems charakterisiert.

Solche »Balanced-Score«-Tests hinsichtlich anderer Parameter sind im Rahmen der medizinschen Diagnostik schon bekannt für die Bewertung des Immunstatus, z.B. bei der Ermittlung der Subpopulationen der T-Lymphozyten (Th1-Th2-Verhältnisse) bei Lepra- oder HIV-Patienten (41,42).

Die folgenden Definitionen für verwendete Begriffe gelten für die gesamte Erfindung und in jeder Kombination.

Lipide im Sinne der Erfindung sind beispielsweise gesättigte und insbesondere einfach sowie vorzugsweise mehrfach ungesättigte Fettsäuren natürlicher oder synthetischer Herkunft (mit mind. 16 Kohlenstoffatomen, z.B. 20 Kohlenstoffatomen) sowie deren natürliche und chemisch/physikalisch/technisch induzierten Derivate und Konstrukte.

Derivate im Sinne der Erfindung (insbesondere in bezug auf die obigen Lipide) sind biologische/natürliche, chemisch induzierte, physikalisch induzierte/synthetisierte Abkömmlinge der Lipide. Diese können durch enzymatische und/oder nichtenzymatische Umsetzungsprozesse aus den Lipiden und/oder den Derivaten entstehen (z.B. Prostaglandin E1, Prostaglandin E2, Prostaglandin E3, Leukotriene, Leukotrien C4 - Leukotrien D4 - Leukotrien D4; HETE (= Hydroxyeisosatetraensäure), PAF (engl. »platelet activating factor« = Thrombozyten-aktivierender Faktor).

Konstrukte im Sinne der Erfindung (insbesondere in bezug auf die obigen Lipide) sind biologisch und/oder chemisch manipulierte Lipide und/oder Derivate unter Hinzufügung oder Entfernung von chemischen/physikalischen/biologischen Strukturen, also Konstrukte, die natürlicherweise nicht existent sind, sondern willentlich durch gerichtete und/oder ungerichtete Modifikation/Synthese in geeigneten Systemen erzeugt werden (z.B. Enzymihibitoren, Enzymaktivatoren, insbesondere Inhibitoren und/oder Aktivatoren der Eicosanoidsynthese wie z.B. Manipulatoren der Cyclooxygenasen, Lipoxygenasen, Rezeptor-Antagonisten, Rezeptor-Agonisten, aber auch für die Detektion geeignete Lipidkonstrukte, die z.B. mittels Fluorometer oder Luminometer oder Massendifferenzmeßapparaturen nachgewiesen und bestimmt werden können).

Organismen im Sinne der Erfindung sind lebende und/oder nicht lebende vielzellige und/oder einzellige Lebensformen wie Menschen, Tiere, Pflanzen, Pilze, Bakterien und/oder Viren und funktionelle Einheiten dieser Lebensformen wie z.B. (aber nicht erschöpfend) Organe, Gewebe, Zellverbände, Zellen, Zellbestandteile (z.B. Mitochondrien).

Proben im Sinne der Erfindung sind manipulierte oder nicht manipulierte Organismen und/oder Teile von/aus einem/mehreren Organismen mit oder ohne vorherige Manipulation des Organismus und/oder der abgegebenen Strukturen/Substanzen (Lipide), sowie Derivate und/oder Konstrukte des/der modulierten und/oder nicht modulierten Organismus/Strukturen/Substanzen (Lipide), die einer Analyse mit dem Ziel zugeführt werden, direkt oder indirekt mindestens zwei Lipidmeßparameter zu erheben. Als weitere Proben können erfindungsgemäß Nukleinsäuren analysiert werden, die im Zusammenhang stehen mit der Regulation/Expression von Lipiden/Eicosanoiden, wie z.B. Synthese- und Abbauenzyme sowie Rezeptoren.

Lipidmeßparameter im Sinne der Erfindung sind qualitativ und/oder quantitativ bestimmbare Einheiten einer Probe (z.B. Lipidmengen, Eicosanoidmengen, Derivatmengen, Konstruktmengen, Enzymmengen, Rezeptormengen, Rezeptordichten, Enzymaktivitäten/ Nukleinsäuremengen, Rezeptorbindungsstärken, Ligandenstabilitäten in einer Probe bzw. einem zu definierenden Umfeld).

Ein Parameter im Sinne der Erfindung ist eine aus einem oder mehreren Lipidmeßparametern abgeleitete Größe für eine direkte oder indirekte Beurteilung bzw. Beschreibung des Zustandes des Lipidverhältnisses der Probe des untersuchten Organismus, auch um daraus die Folgen für den untersuchten Organismus bzgl. dessen weiterer bzw. zu beginnender Modulation vorherzusagen.

Enzyme im Sinne der Erfindung sind Substanzen, die geeignet sind, ein gegebenes Substrat in seiner chemischen/physikalischen/biologischer. Art zu verändern und/oder in chemische/physikalische/biologische Prozesse/Reaktionsabläufe einzugreifen, um dadurch die Subtrate, insbesondere Lipide im Sinne der Erfindung zu verändern (z.B. Cyclooxygenasen, Lipoxygenasen/ Monooxygenasen).

Substrate im Sinne der Erfindung sind Stoffe, die durch Enzyme in ihrer/ihren chemischen/physikalischen/biologischen Eigenschaft/en verändert werden können.

Rezeptoren im Sinne der Erfindung sind Strukturen, die Lipide, Derivate, Konstrukte aber auch Organismen bzw. Proben im Sinne der Erfindung reversibel und/oder irreversibel binden. Dies können natürlicherweise vorkommende Strukturen (z.B. Proteine) aber auch künstlich erzeugte Strukturen/Konstrukte sein, ohne daß sie eine biologische Funktion besitzen müssen (außer, daß sie Liganden binden). Rezeptoren binden Liganden, wobei die Rezeptoren dieselbe, gleiche und/oder ähnliche chemisch/physikalisch/biologisch Struktur aufweisen können wie Liganden (d.h. Prostaglandin E2 kann sowohl ein Ligand als auch ein Rezeptor sein, abhängig davon, wie es sich im biologischen Umfeld/im zu unersuchenden Organismus verhält). Ihre chemische/physikalische/biologische Struktur muß dabei nicht bekannt sein.

Liganden im Sinne der Erfindung sind Strukturen natürlicher und/oder chemisch/physikalisch/biologisch modifizierter natürlicher und/oder künstlich erzeugter Substanzen/Konstrukte, die geeignet sind, an Rezeptoren im Sinne der Erfindung zu binden, und können selber/gleicher/ähnlicher chemischer/physikalischer/biologischer Struktur sein wie die Rezeptoren (z.B. Proteine, Peptide, gesättigte/ungesättigte Fettsäuren und deren Derivate und/oder Konstrukte, Nukleinsäuren und oder Nukleinsäurederivate/Nukleinsäurekonstrukte). Ihre chemische/physikalische/biologische Struktur muß dabei nicht bekannt sein.

Ein »Modulator« bzw. »Effektor« im Sinne der Erfindung ist allgemein ein Stoff oder eine Substanz bzw. Verbindung, die einen oder mehrere Lipidmeßparameter im Sinne der Erfindung verändern kann, z.B. erniedrigen oder erhöhen. Ein Effektor kann also sowohl ein Inhibitor oder Aktivator bzw. ein Antagonist oder Agonist sein.

Allgemein umfaßt das erfindungsgemäße Verfahren folgende Schritte:
(a) Entnahme einer festen, flüssigen oder gasförmigen Probe (z.B. Zellen, Gewebe, Flüssigkeit, Atem/Atemluft) aus einem Organismus (z.B. Mensch/Tier/Pflanze/Bakterium)
(b) Bestimmung der absoluten und relativen Mengen der Lipide/Eicosanoide und/oder z.B. der Regulatoren/Effektoren (z.B. Enzyme/Rezeptoren) dieser Lipide/Eicosanoide
(c) Bestimmung dieser Lipide/Eicosanoide/Enzyme/Rezeptoren im nativen (unmodulierten) und/oder modulierten (stimulierten/inhibierten) Zustand
(d) Differenzierung einer Risikokonstellation (oder z.B. des Gesundheitszustandes/Krankheitszustandes oder einer Sicherung oder eines Ausschlußes bzw. Diagnose) anhand des Verhältnisses aus (a) bis (c) in Gruppen von Organismen/Patienten und/oder Individuen
   sowie gegebenenfalls
(e) Erfassung und Bewertung des Lipid/Eicosanoid/Enzyme/Rezeptorenstatus des zu untersuchenden Organismus und Erfassung und Bewertung des Einflusses von therapeutischen Maßnahmen auf den Organismus (Therapiemonitoring in vitro)
(f) Erfassung und Bewertung (Risikokonstellation) des Einflusses der Lipide/Eicosanoide/Enzyme/Rezeptoren auf komplexe andere Systeme, um Wechelwirkungen zu erkennen (z.B. Schadstoff-modulierte Bakterien oder Gräserpollen und deren Effekte auf die zu untersuchten Proben des zu untersuchenden Organismus)

### Beispiel eines Flußdiagramms der Testdurchführung:

1. Probengewinnung
2. Probenaufbereitung
3. Probenexposition (modulierende Substanz/en)
4. Meßprobengewinnung
5. Meßprobenlagerung
6. Analytik (z.B. durch EIA (engl. »enzyme immuno assay«), RIA (engl. »radio-ligand immuno-sorbent assay«), FIA (engl. »fluorescence immuno assay«), HPLC (engl. »high pressure liquid chromatography«), GC (engl. »gas chromatography«), IH (engl. »immuno histochemistry/immonocytochemistry«), WB (engl. western blotting«), NB (engl. northern blotting«), SB (engl. southern blotting«), GE (engl. »gelelectrophoresis«), PCR (engl. »polymerase chain reaction«))
7. Lipidmeßparametererfassung (semiquantitativ/quantitativ)
8. Meßwertverrechnung
9. Aufstellen der Risikokonstellation (ggf. klinische Interpretation).

Das Lipid/Eicosanoidmuster bzw. -profil (verschiedene Lipide/Eicosanoide/Enzyme/Rezeptoren) kann unter Verwendung beliebiger Proben (fest, flüssig, gasförmig) von einem beliebigen Oganismus bestimmt werden, wobei die angewendete Meß/Analysenmethode keinen besonderen Beschränkungen unterliegt und vom Fachmann nach Maßgabe der praktischen Gegebenheiten gewählt wird.

Die Proben können vor der Analyse nicht moduliert oder moduliert worden sein. Die Modulation kann z.B. erfolgen durch physikalische Mittel (z.B. Wärmestrahlung, radioaktive Strahlung), chemische Stoffe oder Substanzen (z.B. Enzyminhibitoren/-aktivatoren, Rezeptoragonisten/-antagonisten, spezifisch oder unspezifisch) oder biologische Stoffe oder Substanzen (z.B. Schimmelpilze, Baumpollen, Antikörper), die spezifisch oder unspezifisch auf das zu untersuchende System einwirken.

Die Proben können sein/sich befinden in einer biologischen Matrix (Körperflüssigkeiten wie Serum, Plasma, Urin, Stuhl, Atemkondensat oder Liquor; Sekrete der Drüsen von Magen, Darm, Nase, Lunge, Augen; Zellhomogenate; Aerosole; eukaryotische und prokaryotische Zellen, Gewebeverbände und Gewebe) oder in definierten chemischen Lösungen (z.B. Zellkulturmedium, Puffer-/Salz-Lösungen, unphysiologische Lösungen, z.B. in Methanol).

Die Analytik kann quantitativ oder semiquantitativ durchgeführt werden, sie ist jedoch stets geeignet zur Erfassung relativer Unterschiede der Lipide/Eicosanoide/Enzyme/Rezeptoren/Nukleinsäuren entweder manipulierter Proben für einen dieser Parameter untereinander (z.B. unstimulierte Prostaglandin-E2-Synthese zu stimulierter und/oder inhibierter Prostaglandin-E2-Synthese) und/oder zur Erfassung relativer Unterschiede bestimmter im Einzelfall festzulegender Metaboliten zueinander (z.B. Prostaglandin E2 zu Leukotrien D4 jeweils unmoduliert und moduliert).

Wesentlich ist, daß stets mehr als ein Lipidmeßparameter (z.B. vor Behandlung/nach Behandlung oder unmoduliert/moduliert oder Eicosanoid 1/Eicosanoid 2 oder Enzym 1/Enzym 2, Rezeptor 1/Rezeptor 2, Eicosanoid 1/Rezeptor 1, Eicosanoid 1/Enzym 1, Rezeptor 1/Enzym 1, Rezeptor 1/Enzym 2, etc.) von derselben Probe ermittelt wird, die dann für die weitere Auswertung bereitstehen.

Die Lipide/Eicosanoide werden geeigneterweise z.B. mittels Enzym-Immuno-Assay-Technik erfaßt, da mit dieser Technik viele Meßproben am schnellsten und quantitativ gemessen werden können.

Erfaßt werden z.B. Lipide/Eicosanoide (z.B. Leukotriene, Prostaglandine, Prostanoide, Hydroxyeicosatetraensäuren), Lipid/Eicosanoidrezeptoren, Enzyme der Lipid/Eicosanoidsynthese oder die entsprechenden Abbau- oder Regulationsenzyme, sowie dafür kodierende Nukleinsäuren (mRNA).

Zusätzlich zu den bereits bekannten, konventionellen Methoden (s.o.) soll hier ausdrücklich noch auf die Möglichkeit der Lipid/Eicosanoidanalytik mit Hilfe der Microarray- oder Biochiptechnik eingegangen werden. Die Biochip-Technologie ermöglicht die parallele semiquantitative und quantitative Bestimmung einer Vielzahl der oben genannten Lipidmeßparameter, wobei die auf dem Biochip befindlichen Sonden zur Bestimmung der Lipid/Eicosanoidmeßparameter thematisch (in Abhängigkeit von der Untersuchungsfragestellung) gruppiert sein können, oder aber auch entsprechend den gesuchten oder gegebenen Erfordernissen (z.B. abhängig vom Krankheitsbild; bei einem Krankheitsbild ist z.B. pLT und PGE2 wichtig, bei einem anderen pLT und TXB2 oder PGE2 und TXB2 oder PGE2 und PGE2-Rezeptoren und/oder Cyclooxygenase-1) zusammenzustellen bzw. aufzubringen sind. Die physikalischen und/oder chemischen Techniken zur Herstellung von Biochips mit Hilfe bekannter Verfahren unterliegen dabei keinen besonderen Beschränkungen.

Die Visualisierung der nachzuweisenden bzw. zu bestimmenden Lipide/Eicosanoide/Enzyme/Rezeptoren/Nukleinsäuren kann insbesondere mittels Fluorochromen oder phosphoreszierender oder bio/chemolumineszierender oder chromogener Substanzen erfolgen.

Die Erfassung der Analysensignale kann z.B. mittels optischer und/oder elektronischer Meßverfahren (z.B. Potentialänderungen, Leitfähigkeitänderungen) erfolgen.

Ziel der Erfindung ist die Bestimmung von Lipidmeßparameter-Modulations/Effektorquotientenprofilen, um auffällige Zustände (wie z.B. Krankheitsbilder) und Gesundheitszustände und somit Risikokonstellationen zu charakterisieren, oder z.B. nach anti-entzündlichen Stoffen bzw. Substanzen, z.B. Phytopharmaka, zu suchen (»Drugscreening«), oder um die inflammatorische Potenz bei der Bewertung der Biokompatibiliät von implantierbaren Materialien (Produktsicherheit/Patientenschutz) oder die Veranlagung für bestimmte Erkrankungen (Risikokonstellationen) wie Gerinnungsstörungen (z.B. Thrombose, Lungenembolie), Magen-Darm-Erkrankungen (= intestinale Erkrankungen, z.B. Colitis ulcerosa, Morbus Crohn, Ulcus), Nahrungsmittelunverträglichkeiten sowie Entzündungskrankheiten des Gehirns (z.B. Enzephalitis) abschätzen zu können. Weitere Anwendungsmöglichkeiten bestehen in der Geburtsheilkunde, z.B. zur Klärung der Fragestellung, ob ein abnormaler Geburtsverlauf, z.B. wegen abnormaler Wehentätigkeit, zu erwarten ist.

Die Erfindung wird nachfolgend anhand von Beispielen näher erläutert.

### Beispiel 1

Beispielhaft sei hier die Bestimmung des Grundzustandes sowie der stimulierten Zustände von Prostaglandin E2 und Peptid-Leukotrienen bei der Analgetika-Intoleranz geschildert.

Humane Leukozyten des peripheren Blutes werden mit Hilfe eines Dextrangradienten vom Plasma getrennt und auf eine definierte Zellzahl (100.000 Zellen/ml) eingestellt. Anschließend erfolgt die Stimulation durch Inkubation der Zellen, z.B. ohne oder unter Zusatz von Arachidonsäure als modulierende Leitsubstanz, oder anti-IgE als modulierender Effektor für eine definierte Zeit (30 Minuten) bei 37°C in Zellkulturmedium (z.B. RPMI 1640). Nach der Sedimentation der Zellen durch Zentrifugation für 5 Minuten mit 800 x g bei 4°C wird der zellfreie Überstand gesammelt und kann gegebenenfalls bei -80°C unter Stickstoff oder Argon gelagert werden. Anschließend werden diese Meßproben auf eine mit Prostaglandin E2 bzw. Peptid-Leukotrienen beschichtete Polystyrolplatte transferriert und für 18 Stunden bei 4°C unter Zugabe eines anti-Prostaglandin-E2- bzw. anti-Peptid-Leukotrien-Antikörpers inkubiert (»kompetitiver Assay«). Nach einem Waschschritt erfolgt eine Inkubation bei 23°C für 2 Stunden mit einem gegen den ersten Antikörper gerichteten biotinylierten sekundären Antikörper. Nach erneutem Waschen wird mit einer Strepavidinkonjugierten Peroxidase bei 23°C für 1 Stunde inkubiert, und nach erneutem Waschen wird ein Peroxidasesubstrat hinzugegeben und nach ca. 30 Minuten die optische Dichte mittels eines Mehrkanal-Photometers bestimmt. Anhand der mitgeführten Standardkurven können nun die Meßwerte quantitativ bestimmt und zur Berechnung der Lipidmeßparameter-Stimulations/Effektorqoutientenprofile verwendet werden (ein Beispiel für die Berechnung wird weiter unten gegeben).

Ein Beispiel beim Krankheitsbild der Analgetika-Intoleranz ist die verminderte basale Prostaglandin-E2-Synthese (um 20% und mehr), verbunden mit erhöhter basaler Peptid-Leukotrien-Synthese (20% und mehr). Der Quotient Peptid-Leukotrien / Prostaglandin E2 ist kleiner 10). Die Arachidonsäure-induzierte Prostaglandin-E2-Synthese ist unauffällig und die Arachidonsäure-induzierte Peptid-Leukotrien Synthese ist unauffällig bis leicht erhöht (0-40%), wenn diese Werte mit denen von unauffälligen Personen verglichen werden.

Erst die Ermittlung der Lipidmeßparameter-Modulations-/Effektorquotienten ermöglicht eine Differenzierung unterschiedlicher Eicosanoidmuster -bzw. profile, die verschiedenen Risikokonstellationen (wie z.B. bei Patienten mit Asthma bronchiale oder Polyposis nasi et sinuum oder Analgetika-induziertem Asthma) zugeordnet werden können.

Ein geeignetes Verfahren zur Bestimmung von Lipidmeßparametern ist die fluorometrische Messung des Abbaus von ungesättigten Fettsäuren/Arachidonsäure, die mit dem Farbstoff 9-Diethylamino-5H-[alpha]phenoxazin-5-on (38) angefärbt werden. Der Farbstoff dringt in lebende Zellen ein und fluoresziert, solange er intrazellulär an ungesättigte Fettsäuren (d.h. solche mit 2 bis mehr Doppelbindungen) binden kann (39-40). Nach der Aktivierung der Zellen, z.B. durch LPS (Lipopolysaccharid) oder Interleukin-1, kommt es zur Aktivierung von Fettsäure-abbauenden Enzymen (z.B. Phospholipase A2(PLA)), wodurch die Fluoreszenz abnimmt. Demgegenüber kann die Fluoreszenz auch erhöht sein, wenn z.B. endogene Arachidonsäure durch PLA aus Zellmembranen ins Cytoplasma freigesetzt wird, die Degradation derselben jedoch unterbleibt.

9-Diethylamino-5H-[alpha]phenoxazin-5-on wurde bisher lediglich für histologische/cytologische Untersuchungen verwendet, nicht jedoch für quantifizierende Experimente eingesetzt.

Bei Organismen mit veränderter Enzymausstattung kommt es nun zu einem veränderten Abbau der Fettsäuren (schneller oder langsamer), was durch eine Erfassung der Fluoreszenz zu verschiedenen Zeitpunkten (Kinetik) quantitativ gemessen werden kann.

### Beispiel 2

100.000 Leukozyten/ml werden mit einer 10 ⁻⁵ M Lösung von 9-Diethylamino-5H-[alpha]phenoxazin-5-on in PBS-Lösung für 15 min bei Raumtemperatur inkubiert. Anschließend werden sie 2 mal in PBS bei 4°C gewaschen und dann bei 600 x g zentrifugiert und anschließend in eine Reaktionsküvette überführt. Eine Probe wird nun ohne weitere Behandlung fluormetrisch vermessen (Anregungswellenlänge bei 485 nm, Emissionswellenlänge 570 nm). Eine weitere Probe wird z.B. mit LPS (5mg/ml) stimuliert und fluorometrisch vermessen. Die Fluoreszenz beider Proben wird in Zeitabständen von 1 Minute bis zu einer Dauer von 60 Minuten erfaßt. Anschließend kann aus den erhaltenen Werten der Anstieg und der Abfall der Fluoreszenz graphisch aufgetragen werden und die Steigungen der Kurven mittels geeigneter mathematischer Formeln ermittelt werden.

Während der ersten 5-10 Minuten kommt es bei den nicht behandelten Proben zu einem 0-5%igen Anstieg der Fluoreszenz, danach zu einer sigmoid fallenden Fluoreszenz auf 40-60% des Ausgangswertes nach 60 Minuten. Bei den mit LPS-behandelten Proben kommt es innherhalb von 5-10 min zu einem 5-20%igen Anstieg der Fluoreszenz und zu einem 30-80%igen sigmoiden Abfall der Fluoreszenz nach 60 Minuten.

Mit den so erhaltenen Werten können nun normierte Lipidmeßparameter-Modulations/Effektorquotienten erhoben bzw. berechnet und die erhaltenen Profile mit denen von Proben von Referenz-Organismen verglichen werden. Sofern bereits Archivdaten aus vorherigen Untersuchungen vorliegen, können diese verwendet werden.

Durch den Vergleich können dann Risikokonstellationen für den untersuchten Organismus, z.B. hinsichtlich seiner Fähigkeit, ungesättigte Fettsäuren zu metabolisieren, ermittelt werden. Daraus lassen sich dann weitere therapeutische Maßnahmen ableiten.

Diese Methode kann nur in vitro eingesetzt werden. Bisher war eine Anwendung von 9-Diethylamino-5H-[alpha]phenoxazin-5-on für quantitative Bestimmungen des nativen und induzierten Abbaus von ungesättigten Fettsäuren/Arachidonsäure zur Bestimmung von Risikokonstellationen noch nicht bekannt.

Es folgt ein konkretes Berechnungsbeispiel für normierte Lipidmeßparameter-Modulations/Effektorquotienten. Es wird nochmals darauf hingewiesen, daß die Bestimmung von Meßwerten für eine Normgruppe entfallen kann, wenn bereits entsprechende Archivdaten vorliegen.

### Beispiel 3

1) Gewinnung von Leukozyten aus dem peripheren Blut von Untersuchungs-Gruppe 1 / Normgruppe/Untersuchungs-Gruppe 2 mittels Blutabnahme und anschließender Dichtegradientenseparation (3%ige Dextran-Lösung) zur Abtrennung von Plasma und Leukozyten. Die so gewonnenen Leukozyten werden 3 x mit Phosphatpuffer-Lösung (PBS) gewaschen und anschließend in Zellkulturmedium (RPMI 1650) aufgenommen / resuspendiert, worauf eine Zellzahlkonzentration von 100.000 Zellen/ml eingestellt wird (nur bei diesem Beispiel Untersuchungs-Gruppe 2; eine zweite Gruppe ist für den Test vom Prinzip her nicht notwendig, diese dient hier nur zum besseren Verständnis und als zweite Vergleichsgruppe, und zwar zum einen zur Normgruppe und zum anderen zur Untersuchungs-Gruppe).
2) die Zellen werden zu 3 gleichen Teilen (z.B. 3 x je 1 ml Zellsuspension mit 100.000 Zellen/ml) in Reaktionsgefäße (z.B. Kunststoff- oder Glasgefäße) verteilt.
Die erste Teilprobe wird mit einer Kontroll-Lösung versetzt (= ohne weiteren Modulator, = Null- oder Leerwert; Kontroll-Lösung ist das Lösungsmittel, in dem die Modulatoren gelöst werden).
Die zweite Teilprobe wird mit Arachidonsäure versetzt (Konzentration: 10⁻⁵ M; = Leitsubstanz).
Die dritte Teilprobe wird mit einer anti-Immunglobulin-E-Lösung (z.B. 1:100 Verdünnung der von der Fa. DAKO kommerziell erhältlichen anti-human-IgE-Lösung, = Modulator oder Modulationssubstanz) versetzt.
Der Beginn der Exposition / Inkubation mit der Leitsubstanz Arachidonsäure und dem Modulator anti-Immunglobulin-E-Lösung erfolgt zeitgleich bei z.B. 37°C für z.B. 30 Minuten.
Anschließend erfolgt eine Trennung der Zellen vom Zellkultumedium, z.B. durch Zentrifugation bei 4°C und einer Sedimentationsstärke von z.B. 800 x g. Anschließend wird der so erhaltene Überstand der 3 Teilproben, je Teilprobe getrennt, in geeigneten Gefäßen (z.B. Kryoröhrchen) gesammelt und bis zur Analyse mittels EIA bei z.B. -70°C gelagert.
3) In den Zellkulturüberständen der 3 Teilproben werden der Gehalt an Prostaglandin E2 (PGE2) und Peptid-Leukotrienen (pLT) mittels enzymimmunologischer Assays (Nachweisverfahren) (EIA), die spezifisch für PGE2 (= Lipidmeßparameter A) oder pLT (= Lipidmeßparameter B) sind, analysiert und quantifiziert.
Aus der erten Teilprobe erhält man die Wert A₀ (z.B. 350 ±43 pg/ml PGE2) und B₀ (z.B. 120 ±9,7 pg/ml pLT), aus der zweiten Teilprobe erhält man die Werte Aₘₐₓ (z.B. 4120 ±236 pg/ml PGE2 und Bₘₐₓ (z.B. 150 ±10,4 pg/ml pLT), aus der dritten Teilprobe erhält man die Werte A₂ (z.B. 1830 ± 143,8 pg/ml PGE2) und B₂ ( z.B. 83 ±5,7 pg/ml pLT).

**Tabelle 1:**

| Meßwerte (in pg/ml): | | | |
|---|---|---|---|
| Meßparameter | Norm-Gruppe (N) | Untersuchungs-Gruppe 1 (U1) | Untersuchungs-Gruppe 2 (U2) |
| A₀ | 1820±198 | 350±43 | 2172±207 |
| Aₘₐₓ | 4170±275 | 4120±236 | 3976±245 |
| A₂ | 2975±287 | 1830±143 | 785±69 |
| B₀ | 23±3,5 | 120±9,7 | 54±5,8 |
| Bₘₐₓ | 143±12,4 | 150±10,4 | 128±10,4 |
| B₂ | 43±5,7 | 83±5,7 | 135±12,7 |

4) Aus den so gewonnenen Werten der Teilproben erfolgt nun die Berechnung der Modulationsquotienten für den Lipidparameter A (= PGE2) und den Lipidparameter B (=pLT):

**Tabelle 2:**

| Modulationsquotienten: | | | |
|---|---|---|---|
| Meßparameter | Norm-Gruppe (n) | Untersuchungs-Gruppe 1 (u1) | Untersuchungs-Gruppe 2 (u2) |
| Aₘₐₓ/A₀ | 2,3 | 11,8 | 1,8 |
| A₂/A₀ | 1,6 | 5,2 | 0,4 |
| Bₘₐₓ/B₀ | 6,2 | 1,3 | 2,4 |
| B₂/B₀ | 1,9 | 0,7 | 2,5 |
| (auffällige Werte gegenüber der Kontrolle sind in der Tabelle 2 fett markiert) | | | |

(auffällige Werte gegenüber der Kontrolle sind in der Tabelle 2 fett markiert)
Die Modulationsquotienten erlauben eine Unterscheidung der Untersuchungsgruppen (u1, u2) von der Normgruppe (n), nicht aber eine Differenzierung der Untersuchungsgruppen (u1, u2).
5) Normierung durch Division durch die entsprechenden Modulationsquotienten der Normalgruppe (n).

**Tabelle 3:**

| normierte Modulationsquotienten | | | |
|---|---|---|---|
| Meß-parameter | Norm-Gruppe (n) | Untersuchungs-Gruppe 1 (u1) | Untersuchungs-Gruppe 2 (u2) |
| Aₘₐₓ/A₀ | 1 (0,8-1,2) | 5,1 | 0,78 |
| A₂/A₀ | 1 (0,5-1,5) | 3,3 | 0,25 |
| Bₘₐₓ/B₀ | 1 (0,5-1,2) | 0,21 | 0,38 |
| B₂/B₀ | 1 (0,5-1,2) | 0,37 | 1,32 |

(auffällige Werte sind in der Tabelle 3 fett markiert)
Die normierten Modulationsquotienten erlauben ebenfalls nur eine Unterscheidung der Untersuchungsgruppen (u1, u2) von der Normgruppe (n), nicht aber eine Differenzierung der Untersuchungsgruppen (u1, u2). Die Differenzierung der Untersuchungs-Gruppen ist hier abhängig von der Normgruppe, d.h. es können auch zwei oder mehr sonst ähnliche Patienten-Gruppen differenziert werden, z.B. wenn eine »gesunde« Normgruppe nicht erstellt werden kann oder wenn geklärt werden soll, ob sich die beiden Untersuchungsgruppen unterscheiden.
6) Effektorquotienten (Berechnung wie oben)

**Tabelle 4:**

| Effektorquotienten (PGE/pLT): | | | |
|---|---|---|---|
| Meß-parameter | Norm-Gruppe (n) | Untersuchungs-Gruppe 1 (u1) | Untersuchungs-Gruppe 2 (u2) |
| A₀/B₀ | 80,4 | 2,9 | 40,2 |
| Aₘₐₓ /Bₘₐₓ | 29,2 | 27,5 | 31,1 |
| A₂/B₂ | 69,2 | 22,1 | 5,8 |

(auffällige Werte gegenüber der Kontrolle sind in der Tabelle 4 fett markiert)
Die Effektorquotienten erlauben die Differenzierung der beiden Untersuchungs-Gruppen (u1, u2) mit Hilfe des zweiten Modulators (= anti-IgE). Außerdem werden die Unterschiede der beiden Untersuchungs-Gruppen gegenüber der Normgruppe (n) deutlicher.
7) Normierte Effektorquotienten (Berechnung wie oben)

**Tabelle 5:**

| Normierte Effektorquotienten | | | |
|---|---|---|---|
| Meß-parameter | Norm-Gruppe (n) | Untersuchungs-Gruppe 1 (u1) | Untersuchungs-Gruppe 2 (u2) |
| A₀/B₀ | 1 (0,5-1/2) | 0,036 | 0,50 |
| Aₘₐₓ/Bₘₐₓ | 1 (0,9-1,1) | 0,941 | 1,065 |
| A₂/B₂ | 1 (02-1,2) | 0,319 | 0,084 |

(auffällige Werte gegenuber der Kontrolle sind in der Tabelle 5 fett markiert)
Die normierten Effektorquotienten ermöglichen die klare Differenzierung der beiden Untersuchungs-Gruppen (u1, u2) gegenüber der Normgruppe (n), sowie die Differenzierung der beiden Untersuchungs-Gruppen gegeneinander. Hier sind die angegebenen Werte bedingt abhängig von der gewählten Normgruppe, es könnte aber auch eine andere Normgruppe (n-x) gewählt werden. Dies kann z.B. erfolgen, um mehrere Untersuchungsgruppen (u1, u2, u3, u4) gegeneinander zu differenzieren. Wenn keine »Normgruppe« bereitsteht oder erstellt werden kann, kann eine Untersuchungsgruppe (n4) als »Normgruppe« (n-x) ausgewählt werden. Diese Alternative kann auch angewendet werden, wenn z.B. gegenüber »behandelter« (b1) und »anders behandelter« (b2) und/oder »unbehandelter« (b3 = n-x) Gruppe differenziert werden soll.
8) Anmerkung: Die Meßwerte der pLT-Bestimmungen müssen gegebenenfalls mit einem »Ausgleichungsfaktor« (kann experimentell bestimmt werden und liegt zwischen 5 und 100) korrigiert werden, wodurch sich die daraus abgeleiteten/bestimmten Werte relational verändern würden.
9) Schlußfolgerung aus den erhaltenen Ergebnissen:

Die Untersuchungsgruppe 2 wird in die Risikokonstellation 2 eingeteilt, d.h. es liegt eine Analgetika-Intoleranz ohne eine allergische Komponente vor, weshalb eine Desaktivierung gegenüber nichtsteroidalen Analgetika anzuraten ist, und nichtsteroidale Antiphlogistika sollten von Personen dieser Gruppe bis zur erfolgreichen Durchführung der Desaktivierung gemieden werden. Die Untersuchungsgruppe 1 wird der Risikokonstellation 1 zugeordnet, d.h. es liegt eine Analgetika-Intoleranz mit einer deutlichen allergisch-bedingten Kommponente vor, weshalb bei Personen dieser Gruppe eine Allergie-Austestung vorgenommen werden sollte mit gegebenenfalls anschließender Desensibilisierung. Erst danach könnte eine Desaktivierung gegenüber Analgetika sinnvoll sein.

### LITERATUR

### 1) Hirschberg V.G.S.

Mittheilung über einen Fall von Nebenwirkungen des Aspirin. Dt Med Wochenschr 1902;28:416.

### 2) von Euler U.S.

Über die spezifische blutdrucksenkende Substanz des menschlichen Prostata- und Samenblasensekretes. Klin Wschr 1935;14:1182-1186.

### 3) Hanahan D.J.

Platelet activating factor: A biological active phosphoglyceride. Ann Rev Biochem 1986; 55: 483-509.

### 4) Hamberg M., Svensson J., Samuelsson B.

Thromboxanes: a new group of biologically active compounds derived from prostaglandin endoperoxidase. Proc Natl Acad Sci USA 1975; 72:2994-2998.

### 5) Vane J.R.

Inhibition of prostaglandin biosynthesis as a mechanism of action for aspirin-like drugs. Nature (New Biol) 1971; 231:232-235.

### 6) Bergström S.

Prostaglandines: Members of a new hormonal system. Science 1967; 157:382-390.

### 7) Samuelsson B.

Some recent advances in leukotriene research. Adv Exp Med Biol 1997; 433:1-7.

### 8) Samuelsson B., Dahlen S.-E., Lindgren J.A. Rouzer C.A, Serhan C.N.

Leukotrienes and lipoxins: structures, biosynthesis, and biological effects. Science 1987;237:1171-1176.

### 9) Holzman J.Y.

Arachidonic acid metabolism. Am Rev Respir Dis 1991; 143:188-203.

### 10) Smith W.L.

The eicosanoids and their biochemicaal mechanisms of action. Biochem J 1989; 259;315-324.

### 11) Fradin A., Zirrolli J.A., Maclouf J., Vausbinder L., HENSON P.M., Murphy R.C.

Platelet-activating factor and leukotriene biosynthesis in whole blod - a model for the study of transcellular arachidonic metabolism.
J Immunol 1989;143;3680-3685.

### 12) Smith D.L & Willis A.L.

A sugested shorthand nomenclature for the eicosanoids.
Lipids 1987;22:983-987.

### 13) Corey E.J., Niwa H., Falck J.R., Mioskowski C., Arai Y., Marfat A.

Recent studies on the chemical synthesis of eicosanoids.
Adv Prostaglandin Thromboxane Res 1980;6:19-25.

### 14) Willis A.L. & Smith D.L.

Metabolism of arachidonic acid.
in: The handbook of immunopharmacology. Lipid mediators, ed.: Cunningham F.M., Academic Press, London, 1994:1-32.

### 15) Slater T.F. & McDonald-Gibson R.G.

Introduction to the eicosanoids.
in: Prostaglandins and related substances, eds.: Benedetto C, McDonald-Gibson RG, Nigam S, Slater TF, IRL Press, Oxford, 1987:1-4.

### 16) Rowley A.F., Kuhn H., Schewe T.

Eicosanoids and related compounds in plants and animals.
PoPrinceton University Press, Princeton, 1998.

### 17) Stanley-Samuelson D.W..

Physiological role of prostaglandins and other eicosanoids in invertebrates.
Biol Bull 1987;173:92-109.

### 18) Bell M.V, Henderson R.J., Sargent J.R.

The role of polyansaturated fatty acid in fish.
Comp Biochem Physiol 1986;85B:711-719.

### 19) Vick B.A.

Oxygenated fatty acids of the lipoxygenase pathway.
Chapter 5
in: Lipid metabolism in plants.
ed: Moore T.S.
CRC Press, Boca Raton 1993:167-191.

### 20) Dennis E.A.

Diversity of group types, regulation, and function of phospholipase A2.
J Biol Chem 1994; 269:13057-13063.

### 21) Creminon C., Habib A., Maclouf J., Pradelles P., Grassi J.

Differential measurement of constitutive (COX-1) and inducable (COX-2) cyclooxygenase expression in human umbilical cells using specific immunometric enzyme immunoassays.
Biochim Biophys Acta 1995;1254:341-348.

### 22) O'Neil G.P. & Ford-Hutshinson A.W.

Expression of mRNA for cyclooxygenase-1 and cyclooxygenase 2 in human tissue.
FEBS Lett 1993;330:156-160.

### 23) Kennedy I., Coleman R.A., Humphrey P.P.A., Levy H.P., Lumley P.

Studies on the characterisation of prostanoid receptors: a proposed classification
Prostaglandins, 1982: 24:667-689.

### 24) DeWitt D.L & Meade E.A.

Serum and glucocorticoid regulation of gene transcription and expresssion of prostaglandin H synthase-1 and prostaglandin H synthase-2 isoenzymes.
Arch Biochem Biophys 1993; 306:94-102.

### 25) Gardiner P.J. Abram T.S. Tudhope S.R. Cuthbest N.J. Norman P., Brink C.

Leukotriene receptors and their selective antagonists.
Adv Prstaglandin Thromboxane Leukot Res 1994; 2:49-61.

### 26) Hong J.L. & Lee L.-Y.

Cigaret smoke-induced bronchoconstriction: causative agents and role of thromboxane receptors.
J Appl Physiol 1995; 78:2260-2266.

### 27) Coleman R.A., Smith W.L., Narumiya s.

Classification of the prostanoid receptors: properties, distribution, and structure of the receptors and thier subtypes. Pharmacol rev. 1994;46:205-229.

### 28) Taylor G.W. & Wellings R.

Measurements of fatty acids and their metabolites.
in: Lipid mediators
ed.: Cunningham F.M.
Accademic Press, London 1994:33-60.

### 29) Kulmacz R.J., & Lands W.E.M.

Cyclooxygenase: Measurement, purification and properties.
in: Prostaglandins and related substances.
eds.: Benedetto C., McDonald-Gibson R.G., Nigam S., Slater T.F.
Oxofrd University Press, Oxford 1989:209-228.

### 30) Schewe T., Kühn H., Rapoport S.M.

Lipoxygenases: measurement, charaterization, and properties.
in: Prostaglandins and related substances.
eds.: Benedetto C., McDonald-Gibson R.G., Nigam S., Slater T.F.
Oxofrd University Press, Oxford 1989:229-242.

### 31) Vilaseca J., Salas A., Guarner F., Rodriguez R., Malagelada J.R.

Participation of thromboxane and other eicosanoid synthesis in the course of experimental inflammatory colitis.
Gastroenterology 1990; 98:269-277.

### 32) De Weck A.L., Stadler B.M., Urwyler A., Wehner H.U., Bühlmann R.P.

Cellular antigen stimulation test (CAST) - A new dimension in allergy diagnostics.
Allergy Clin Immunol News 1993; 5:9-14.

### 33) Baenkler H.-W., Schäfer D., Hosemann W.

Eicosanoids from biopsies of normal and polypous nasal mucosa.
Rhinology 1996; 34:166-170.

### 34) Schäfer D., Lindenthal U., Wagner M., Bölcskei P.L., Baenkler H.W.

Effect of prostaglandin E2 on eicosanoid release by human bronchial biopsy specimens from normal and inflamed mucosa. Thorax 1996; 51:919-932.

### 35) Sauer S.K., Schäfer D., Kress M., Reeh P.W.

Stimulated prostaglandin E2 from rat skin, in vitro.
Life Science 1998; 62: 2045-2055.

### 36) Schäfer D., Schmid M., Göde U., Baenkler H.-W.

Dynymics of eicosanoids in peripheral blood cells during bronchial provocation in aspirin-intolerant asthmatics.
Eur Respir J 1999; 13:638-646.

### 37) Westcott J.Y.

The measurement of leukotrienes in human fluids.
Clin Rev Allergy Immnunol 1999;17:153-177.

### 38) Greenspan P., Mayer E.P., Fowler S.D.

Nile Red: a selective fluorescent stain for intrecellular lipid droplets.
J Cell Biol. 1985; 100:965-973.

### 39) Dvorack A.M., Dvorak H.F., Peters S.P., Shulman E.S., Mac Glashan D.W., Pyne K., Harvey V.S., Galli S.J., LichtensteinL.M.

Lipid bodies: cytoplasmatic organelles important to arachidonate metabolism in macrophages and mast cells.
J Immunol 1983; 131:2965-2976.

### 40) Weller P.F. & Dvorack A.M.

Arachidonic acid incorporation by cytoplasmatic lipid bodies of human eosinophils.
Blood 1985; 65:1269-1274.

### 41) Erdmann E.

Klinische Kardiologie.
Springer Verlag, Berlin, 2000.

### 42) Adler G., Beglinger C., Müller-Lissner S., Schmigel W.

Klinische Gastroenterologie und Stoffwechsel.
Springer Verlag, Berlin, 2000.

## Patentansprüche

1. Verfahren zur Diagnose von Krankheitszuständen oder Prädispositionen dafür auf der Grundlage von Lipidmeßparameter-Modulations/Effektorquotientenprofilen, bei dem
(a) eine aus einem zu untersuchenden Organismus entnommene Probe in soviele gleiche Teilproben aufgeteilt wird, daß für jeden Lipidmeßparameter A, B, C ... ein Nullwert A₀, B₀, C₀, ... in Abwesenheit eines modulierenden Effektors; ein Wert für eine Leitsubstanz (Leitwert) Aₘₐₓ, Bₘₐₓ/ Cₘₐₓ, ... in Gegenwart eines modulierenden Effektors/einer modulierenden Leitsubstanz; und mindestens ein Wert für eine weitere Modulation A₂, B₂, C₂, ... in Gegenwart eines weiteren modulierenden Effektors gemessen werden kann,
(b) für jeden Lipidmeßparameter A, B, C, ... der Probe aus dem zu untersuchenden Organismus
(i) die Quotienten der Meßwerte Aₘₐₓ/A₀, A₂/A₀; Bₘₐₓ/B₀, B₂/B₀; Cₘₐₓ/C₀, C₂/C₀; ... gebildet und anschließend die für den zu untersuchenden Organismus erhaltenen Werte durch die entsprechenden Werte einer oder mehrerer Normgruppe(n) dividiert werden, wodurch sich normierte Modulationsquotienten ergeben, die in ihrer Gesamtheit ein normiertes Modulationsquotientenprofil für den zu untersuchenden Organismus bilden, und
(ii) aus den Nullwerten A₀, B₀, C₀ ... Quotienten A₀/B₀, B₀/A₀, A₀/C₀, C₀/A₀, B₀/C₀, C₀/B₀ ... in beliebiger Kombination gebildet werden; aus den Werten für eine Leitsubstanz Aₘₐₓ, Bₘₐₓ, Cₘₐₓ ... Quotienten Aₘₐₓ/Bₘₐₓ, Bₘₐₓ/Aₘₐₓ, Aₘₐₓ/Cₘₐₓ, Cₘₐₓ/Aₘₐₓ, Bₘₐₓ/Cₘₐₓ, Cₘₐₓ/Bₘₐₓ ... in beliebiger Kombination gebildet werden; und aus den Werten für mindestens eine weitere Modulation A₂, B₂, C₂ ... Quotienten A₂/B₂, B₂/A₂, A₂/C₂, C₂/A₂, B₂/C₂, C₂/B₂ ... in beliebiger Kombination gebildet werden; und anschließend die für den zu untersuchenden Organismus erhaltenen Werte durch die entsprechenden für die Normgruppe(n) erhaltenen Werte dividiert werden, wodurch sich normierte Effektorquotienten ergeben, die in ihrer Gesamtheit ein normiertes Effektorquotientenprofil für den zu untersuchenden Organismus bilden,
und
(c) eine Risikokonstellation, ein Krankheitszustand oder eine Prädispositionen dafür durch den Vergleich der normierten Modulations- und Effektorquotientenprofile des zu untersuchenden Organismus mit denen einer entsprechenden Untersuchungsgruppe, bei der die interessierende Risikokonstellation, der interessierende Krankheitszustand oder die interessierende Prädisposition vorliegt, gesichert oder ausgeschlossen wird.

2. Verfahren nach Anspruch 1, wobei in Schritt (a) die Lipidmeßparameter unter Meßparametern für ungesättigte Fettsäuren, Abbau- und Syntheseenzyme für ungesättigte Fettsäuren, sowie dafür kodierende Nukleinsäuren, und unter solchen für Rezeptoren für ungesättigte Fettsäuren, sowie dafür kodierende Nukleinsäuren, ausgewählt werden.

3. Verfahren nach Anspruch 2, wobei die ungesättigten Fettsäuren unter dem Thrombozyten-aktivierenden Faktor und den Eicosanoiden ausgewählt werden.

4. Verfahren nach Anspruch 3, wobei die Eicosanoide unter den Peptid-Leukotrienen, Prostaglandin E2, Thromboxan A2 und Thromboxan B2 ausgewählt werden.

5. Verfahren nach einem der vorstehenden Ansprüche, wobei in dem in Anspruch 1 definierten Schritt (a) als stimulierende Leitsubstanz der Effektor Arachidonsäure oder ein chemotaktisches Peptid verwendet wird.

6. Verfahren nach einem der vorstehenden Ansprüche, wobei in dem in Anspruch 1 definierten Schritt (a) als weiterer modulierender Effektor ein Stoff verwendet wird, der einen Krankheitszustand hervorrufen kann oder an dessen Entstehung oder Entwicklung beteiligt ist.

7. Verfahren nach einem der vorstehenden Ansprüche, wobei der Krankheitszustand ausgewählt ist unter Tumoren, cystischer Fibrose, Polyposis, Asthma bronchiale, einer Unverträglichkeit, Gerinnungsstörungen, Infektbewältigung und einer Entzündung.

8. Verfahren nach Anspruch 7, wobei die Unverträglichkeit eine Nahrungsmittel-, Nahrungsmittelzusatzstoff- oder Arzneimittelunverträglichkeit oder eine Allergie ist, und wobei die Gerinnungsstörungen die Basis für Thrombosen oder Blutungen oder Thrombophilie darstellen, und wobei die Infektbewältigung eine Abwehr bakterieller, viraler oder mykotischer Elemente, z.B. bei bakterieller, viraler oder mykotischer Mucositis, ist, und wobei die Entzündung Enzephalitis, Sinusitis, Rhinitis, Neurodermitis, Morbus Crohn oder Colitis ulcerosa ist.

9. Verfahren nach Anspruch 8, wobei die Arzneimittelunverträglichkeit eine Analgetikaunverträglichkeit und die Allergie eine Pollen-, Sporen-, Milben-, Wespen- oder Bienengiftallergie ist.

10. Verfahren nach Anspruch 9, wobei die Analgetikaunverträglichkeit Acetylsalicylsäureunverträglichkeit ist.

11. Verfahren nach einem der Ansprüche 2 bis 10, wobei zur Bestimmung der Lipidmeßparameter ein oder mehrere, gegebenenfalls markierte(s) Eicosanoid(e) oder der Farbstoff 9-Diethylamino-5H-[alpha]phenoxazin-5-on verwendet wird/werden.

12. Verfahren nach einem der Ansprüche 2 bis 10, wobei zur Bestimmung der Lipidmeßparameter immobilisierte Sonden verwendet werden, die ausgewählt sind unter Antikörpern oder funktionellen Fragmenten davon gegen Abbau- oder Syntheseenzyme von ungesättigten Fettsäuren oder gegen Rezeptoren für ungesättigte Fettsäuren; Nukleinsäuren, die an Nukleinsäuren hybridisieren, die für Abbau- oder Syntheseenzyme von ungesättigten Fettsäuren oder für Rezeptoren für ungesättigte Fettsäuren kodieren.

13. Verfahren nach Anspruch 12, wobei die Antikörper unter polyklonalen, monoklonalen und Single-chain-Antikörpern, und die Nukleinsäuren unter cDNA, mRNA und Oligonukleotiden ausgewählt werden.

14. Verfahren nach Anspruch 12 oder 13, wobei die immobilisierten Sonden ein adressierbares Muster auf einer Oberfläche bilden.

15. Verfahren zur Überwachung des Verlaufs von Therapien von Krankheitszuständen auf der Grundlage von Lipidmeßparameter-Modulations/Effektorquotientenprofilen, bei dem ein Verfahren nach einem der Ansprüche 1 bis 14 nach der Verabreichung oder in Gegenwart eines geeigneten Medikaments durchgeführt wird.

16. Verfahren zum Auffinden von Wirkstoffen zur Behandlung von Krankheitszuständen auf der Grundlage von Lipidmeßparameter-Modulations/Effektorquotientenprofilen, bei dem ein Verfahren nach einem der Ansprüche 1 bis 14 nach der Verabreichung oder in Gegenwart eines Kandidatenwirkstoffs durchgeführt wird.

17. Verfahren zum Auffinden von Stoffen, die in der Lage sind, einen Krankheitszustand gemäß einem der Ansprüche 7 bis 10 auszulösen, auf der Grundlage von Lipidmeßparameter-Modulations/Effektorquotientenprofilen, bei dem ein Verfahren nach einem der Ansprüche 1 bis 14 nach der Verabreichung/Applikation oder in Gegenwart eines solchen Stoffes durchgeführt wird.

18. Meßinstrument zur Durchführung eines Verfahrens nach einem der Ansprüche 1 bis 17, das eine Oberfläche aufweist, auf der zur Bestimmung der Lipidmeßparameter definierte Sonden immobilisiert sind, die unter Antikörpern oder funktionellen Fragmenten davon . gegen Abbau- oder Syntheseenzyme von ungesättigten Fettsäuren oder gegen Rezeptoren für ungesättigte Fettsäuren, sowie unter Nukleinsäuren ausgewählt sind, die an Nukleinsäuren hybridisieren, welche für Abbau- oder Syntheseenzyme von ungesättigten Fettsäuren oder für Rezeptoren für ungesättigte Fettsäuren kodieren, wobei die Antikörper vorzugsweise unter polyklonalen, monoklonalen und Single-chain-Antikörpern, und die Nukleinsäuren vorzugsweise unter cDNA, mRNA und Oligonukleotiden ausgewählt sind.

19. Meßinstrument nach Anspruch 18, wobei die Sonden ein adressierbares Muster auf der Oberfläche bilden.

## Claims

1. Method for the diagnosis of pathological states or predispositions thereto based on lipid measurement parameter modulation/effector quotient profiles, in which
(a) a sample taken from an organism to be investigated is divided into sufficient equal part-samples to allow measurement for each lipid measurement parameter A, B, C ... of a zero value A₀, B₀, C₀, ... in the absence of a modulating effector; of a value for an indicator substance (indicator value) Aₘₐₓ, Bₘₐₓ, Cₘₐₓ, ... in the presence of a modulating effector/of a modulating indicator substance; and of at least one value for a further modulation A₂, B₂, C₂, ... in the presence of a further modulating effector,
(b) for each lipid measurement parameter A, B, C, ... of the sample from the organism to be investigated
(i) the quotients of the measurements Aₘₐₓ/A₀, A₂/A₀; BₘₐₓB₀, B₂/B₀; Cₘₐₓ/C₀, C₂/C₀; ... are formed and then the values obtained for the organism to be investigated are divided by the corresponding values of one or more standard group(s), resulting in standardized modulation quotients which in their totality form a standardized modulation quotient profile for the organism to be investigated, and
(ii) quotients A₀/B₀, B₀/A₀, A₀/C₀, C₀/A₀, B₀/C₀, C₀/B₀ ... in any combination are formed from the zero values A₀, B₀, C₀ ... ; quotients Aₘₐₓ/Bₘₐₓ, Bₘₐₓ/Aₘₐₓ, Aₘₐₓ/Cₘₐₓ, Cₘₐₓ/Aₘₐₓ, Bₘₐₓ/Cₘₐₓ, Cₘₐₓ/Bₘₐₓ ... are formed in any combination from the values for an indicator substance Aₘₐₓ, Bₘₐₓ, Cₘₐₓ ...; and quotients A₂/B₂, B₂/A₂, A₂/C₂, C₂/A₂, B₂/C₂, C₂/B₂ ... are formed in any combination from the values for at least one further modulation A₂, B₂, C₂ ...; and then the values obtained for the organism to be investigated are divided by the corresponding values obtained for the standard group (s), resulting in standardized effector quotients which in their totality form a standardized effector quotient profile for the organism to be investigated,
and
(c) a constellation of risk factors, a pathological state or a predisposition thereto is confirmed or excluded by comparing the standardized modulation and effector quotient profiles of the organism to be investigated with those of a corresponding investigation group in which the constellation of risk factors of interest, the pathological state of interest or the predisposition of interest is present.

2. Method according to Claim 1, where in step (a) the lipid measurement parameters are selected from measurement parameters for unsaturated fatty acids, degrading enzymes and synthesizing enzymes for unsaturated fatty acids, and nucleic acids coding therefor, and from those for receptors for unsaturated fatty acids, and nucleic acids coding therefor.

3. Method according to Claim 2, where the unsaturated fatty acids are selected from platelet-activating factor and eicosanoids.

4. Method according to Claim 3, where the eicosanoids are selected from peptide leukotrienes, prostaglandin E2, thromboxane A2 and thromboxane B2.

5. Method according to any of the preceding claims, where the stimulating indicator substance used in step (a) defined in Claim 1 is the effector arachidonic acid or a chemotactic peptide.

6. Method according to any of the preceding claims, where the further modulating effector used in step (a) defined in Claim 1 is a substance which may cause a pathological state or is involved in the onset or development thereof.

7. Method according to any of the preceding claims, where the pathological state is selected from tumours, cystic fibrosis, polyposis, bronchial asthma, an intolerance, coagulation defects, overcoming of infection, and an inflammation.

8. Method according to Claim 7, where the intolerance is a food, food additive or drug intolerance or an allergy, and where the coagulation defects represent the basis for thromboses or haemorrhages or thrombophilia, and where the overcoming of infection is a resistance to bacterial or viral or mycotic elements, e.g. associated with bacterial, viral or mycotic mucositis, and where the inflammation is encephalitis, sinusitis, rhinitis, neurodermatitis, Crohn's disease or ulcerative colitis.

9. Method according to Claim 8, where the drug intolerance is an analgesic intolerance and the allergy is a pollen, spore, mite, wasp or bee venom allergy.

10. Method according to Claim 9, where the analgesic intolerance is intolerance of acetylsalicylic acid.

11. Method according to any of Claims 2 to 10, where one or more, optionally labelled eicosanoid(s) or the dye 9-diethylamino-5H-[alpha]phenoxazin-5-one is/are used to determine the lipid measurement parameters.

12. Method according to any of Claims 2 to 10, where immobilized probes selected from antibodies or functional fragments thereof against degrading enzymes or synthesizing enzymes of unsaturated fatty acids or against receptors for unsaturated fatty acids; nucleic acids which hybridize onto nucleic acids which code for degrading enzymes or synthesizing enzymes of unsaturated fatty acids or for receptors for unsaturated fatty acids, are used to determine the lipid measurement parameters.

13. Method according to Claim 12, where the antibodies are selected from polyclonal, monoclonal and single-chain antibodies, and the nucleic acids are selected from cDNA, mRNA and oligonucleotides.

14. Method according to Claim 12 or 13, where the immobilized probes form an addressable pattern on a surface.

15. Method for monitoring the course of therapies of pathological states based on lipid measurement parameter modulation/effector quotient profiles, in which a method according to any of Claims 1 to 14 is carried out after the administration or in the presence of a suitable medicament.

16. Method for finding active substances for the treatment of pathological states based on lipid measurement parameter modulations/effector quotient profiles, in which a method according to any of Claims 1 to 14 is carried out after the administration or in the presence of a candidate active substance.

17. Method for finding substances able to induce a pathological state according to any of Claims 7 to 10, based on lipid measurement parameter modulations/effector quotient profiles, in which a method according to any of Claims 1 to 14 is carried out after the administration/application or in the presence of such a substance.

18. Measuring instrument for carrying out a method according to any of Claims 1 to 17, which has a surface on which probes defined for determination of the lipid measurement parameters are immobilized, which probes are selected from antibodies or functional fragments thereof against degrading enzymes or synthesizing enzymes of unsaturated fatty acids or against receptors for unsaturated fatty acids, and from nucleic acids which hybridize onto nucleic acids which code for degrading enzymes or synthesizing enzymes of unsaturated fatty acids or for receptors for unsaturated fatty acids, where the antibodies are preferably selected from polyclonal, monoclonal and single-chain antibodies, and the nucleic acids are preferably selected from cDNA, mRNA and oligonucleotides.

19. Measuring instrument according to Claim 18, where the probes form an addressable pattern on the surface.

## Revendications

1. Procédé pour diagnostiquer des états pathologiques ou des prédispositions à la maladie sur la base de profils de quotients de modulation / effecteurs de paramètres de mesure de lipides, selon lequel
(a) un échantillon prélevé sur l'organisme à examiner est réparti en autant d'échantillons partiels semblables pour que puissent être mesurées pour chaque paramètre de mesure de lipides A, B, C ... une valeur zéro A₀, B₀, C_{0,} ... en l'absence d'un effecteur modulant ; une valeur pour une substance indicatrice (valeur indicatrice) Aₘₐₓ, Bₘₐₓ, Cₘₐₓ, ... en présence d'un effecteur modulant / d'une substance indicatrice modulante ; et au moins une valeur pour une autre modulation A₂, B₂, C₂, ... en présence d'un autre effecteur modulant,
(b) pour chaque paramètre de mesure des lipides A, B, C, ... de l'échantillon prélevé sur l'organisme à examiner,
(i) on forme les quotients des valeurs mesurées Aₘₐₓ/A₀, A₂/A₀ ; Bₘₐₓ/B₀, ^{B}2/^{B}0 ; Cₘₐₓ/Co, C₂/C₀ ; ... puis divise les valeurs obtenues pour l'organisme à examiner par les valeurs correspondantes d'un ou plusieurs groupes de référence, obtenant ainsi des quotients de modulation normés qui dans leur totalité forment pour l'organisme à examiner un profil de quotients de modulation normé, et
(ii) à partir des valeurs zéro A₀, B₀, C₀ ..., on forme des quotients A₀/B₀, B₀/A₀, A₀/C₀, C₀/A₀, B₀/C₀, C₀/B₀ ... dans une combinaison quelconque ; à partir des valeurs pour une substance indicatrice Aₘₐₓ, Bₘₐₓ, Cₘₐₓ ..., on forme des quotients Aₘₐₓ/Bₘₐₓ, Bₘₐₓ/Aₘₐₓ, Aₘₐₓ/Cₘₐₓ, Cₘₐₓ/Aₘₐₓ, Bₘₐₓ/Cₘₐₓ, Cₘₐₓ/Bₘₐₓ ... dans une combinaison quelconque ; et à partir des valeurs pour au moins une autre modulation A₂, B₂, C₂ ..., on forme des quotients A₂/B₂, B₂/A₂, A₂/C₂, C₂/A₂, B₂/C₂, C₂/B₂ ... dans une combinaison quelconque ; et on divise ensuite les valeurs obtenues pour l'organisme à examiner par les valeurs correspondantes obtenues pour le ou les groupes de référence, obtenant ainsi des quotients effecteurs normés qui dans leur totalité forment pour l'organisme à examiner un profil de quotients effecteurs normé,
et
(c) on établit ou exclut une constellation de risques, un état pathologique ou une prédisposition à la maladie en comparant les profils normés de quotients de modulation et effecteurs de l'organisme à examiner avec ceux d'un groupe de contrôle correspondant pour lequel la constellation de risques intéressante, l'état pathologique intéressant ou la prédisposition intéressante existe.

2. Procédé selon la revendication 1, les paramètres de mesure des lipides pour l'étape (a) étant choisis parmi les paramètres de mesure pour des acides gras insaturés, des enzymes de dégradation et de synthèse des acides gras insaturés, ainsi que des acides nucléiques codant pour cela, et entre autres pour des récepteurs pour acides gras insaturés ainsi que des acides nucléiques codant pour cela.

3. Procédé selon la revendication 2, les acides gras insaturés étant choisis parmi le facteur activant des thrombocytes et les eicosanoïdes.

4. Procédé selon la revendication 3, les eicosanoïdes étant choisis parmi les leucotriènes peptidiques, la prostaglandine E2, le thromboxane A2 et le thromboxane B2.

5. Procédé selon l'une des revendications précédentes, l'effecteur acide arachidonique ou un peptide chimiotactique étant utilisé comme substance indicatrice stimulante dans l'étape (a) définie dans la revendication 1.

6. Procédé selon l'une des revendications précédentes, une substance pouvant provoquer un état pathologique ou contribuant à son apparition ou à son évolution étant utilisée comme autre effecteur modulant dans l'étape (a) définie dans la revendication 1.

7. Procédé selon l'une des revendications précédentes, l'état pathologique étant choisi parmi les tumeurs, la fibrose kystique, la polypose, l'asthme bronchique, une intolérance, des troubles de la coagulation, une lutte contre une maladie et une inflammation.

8. Procédé selon la revendication 7, l'intolérance étant une intolérance à un aliment, à un additif alimentaire ou à un médicament ou une allergie, et les troubles de la coagulation représentant l'origine de thromboses ou d'hémorragies ou de thrombophilie, et la lutte contre une maladie étant une défense contre des éléments bactériens, viraux ou mycosiques, par exemple en cas de mucosite bactérienne, virale ou mycosique, et l'inflammation étant une encéphalite, une sinusite, une rhinite, une neurodermite, la maladie de Crohn ou une colite ulcéreuse.

9. Procédé selon la revendication 8, l'intolérance médicamenteuse étant une intolérance aux analgésiques et l'allergie une allergie aux pollens, aux spores, aux acariens, au venin de guêpe ou d'abeille.

10. Procédé selon la revendication 9, l'intolérance aux analgésiques étant une intolérance à l'acide acétylsalicylique.

11. Procédé selon l'une des revendications 2 à 10, un ou plusieurs eicosanoïdes, marqués le cas échéant, ou le colorant 9-diéthylamino-5H[alpha]phénoxazinone-5 étant utilisés pour déterminer les paramètres de mesure des lipides.

12. Procédé selon l'une des revendications 2 à 10, des sondes immobilisées étant utilisées pour déterminer les paramètres de mesure des lipides, ces sondes étant choisies parmi des anticorps ou leurs fragments fonctionnels agissant contre des enzymes de dégradation ou de synthèse d'acides gras insaturés ou contre des récepteurs pour acides gras insaturés ; des acides nucléiques hybridant avec des acides nucléiques qui codent pour des enzymes de dégradation ou de synthèse des acides gras insaturés ou pour des récepteurs pour acides gras insaturés.

13. Procédé selon la revendication 12, les anticorps étant choisis parmi des anticorps polyclonaux, monoclonaux et à chaîne unique et les acides nucléiques parmi l'ADNc, l'ARNm et les oligonucléotides.

14. Procédé selon la revendication 12 ou 13, les sondes immobilisées formant un échantillon adressable sur une surface.

15. Procédé pour observer le déroulement de thérapies contre des états pathologiques sur la base de profils de quotients de modulation / effecteurs de paramètres de mesure de lipides, dans lequel un procédé selon l'une des revendications 1 à 14 est réalisé après l'administration ou en présence d'un médicament approprié.

16. Procédé pour détecter des substances actives pour le traitement d'états pathologiques sur la base de profils de quotients de modulation / effecteurs de paramètres de mesure de lipides, dans lequel un procédé selon l'une des revendications 1 à 14 est réalisé après l'administration ou en présence d'une substance active candidate.

17. Procédé pour détecter des substances qui sont aptes à déclencher un état pathologique selon l'une des revendications 7 à 10 sur la base de profils de quotients de modulation / effecteurs de paramètres de mesure de lipides, dans lequel un procédé selon l'une des revendications 1 à 14 est réalisé après l'administration /l'application ou en présence d'une telle substance.

18. Instrument de mesure pour la réalisation d'un procédé selon l'une des revendications 1 à 17, lequel présente une surface sur laquelle sont immobilisées des sondes définies pour la détermination de paramètres de mesure de lipides, lesquelles sont choisies parmi des anticorps ou leurs fragments fonctionnels agissant contre des enzymes de dégradation ou de synthèse d'acides gras insaturés ou contre des récepteurs pour acides gras insaturés, ainsi que parmi des acides nucléiques hybridant avec des acides nucléiques qui codent pour des enzymes de dégradation ou de synthèse des acides gras insaturés ou pour des récepteurs pour acides gras insaturés, les anticorps étant choisis de préférence parmi des anticorps polyclonaux, monoclonaux et à chaîne unique, et les acides nucléiques de préférence parmi l'ADNc, l'ARNm et des oligonucléotides.

19. Instrument de mesure selon la revendication 18, les sondes formant un échantillon adressable sur la surface.
